(19) Europäisches Patentamt
European Patent Office
Office européen des brevets

(11) Publication number: **0 468 114 A2**

(12) # EUROPEAN PATENT APPLICATION

(21) Application number: **90310534.4**

(51) Int. Cl.⁵: **A61L 15/28**

(22) Date of filing: **26.09.90**

(30) Priority: **24.07.90 CN 90104824**

(43) Date of publication of application: **29.01.92 Bulletin 92/05**

(84) Designated Contracting States: **DE ES GB**

(71) Applicant: **CHINA NATIONAL CHEMICALS IMPORT & EXPORT CORPORATION**
**Er Li Gou, Xi Jiao**
**Beijing(CN)**

Applicant: **BEIJING TEXTILE RESEARCH INSTITUTE**
**No. 10 Tian Shui Yuan Dong Jie, Jin Tai Lu**
**Chao Yang District, Beijing(CN)**

(72) Inventor: **Xue, Digeng**
**No. 10 Tian Shui Yuan Dong Jie, Jin Tai Lu**
**Chao Yang District, Beijing(CN)**

(74) Representative: **Pendlebury, Anthony et al**
**PAGE, WHITE & FARRER 54 Doughty Street**
**London WC1N 2LS(GB)**

(54) **Soluble haemostatic fabric.**

(57) The invention reltes to the soluble hemostatic fabrics having Formula (I) or Formula (II), and to a process for preparing them. The hemostatic fabrics of the present invention may dissolve at the wound or be absorbed by body fluid while contacting the wound, and especially have good hemostatic effect for the patients who suffer from the disturbance of blood coagulation. The soluble hemostatic fabrics of the present invention have broad application in any type of surgery and other situations where the control of bleeding is required, and can be prepared by simple and enconomic process.

Fig. 1

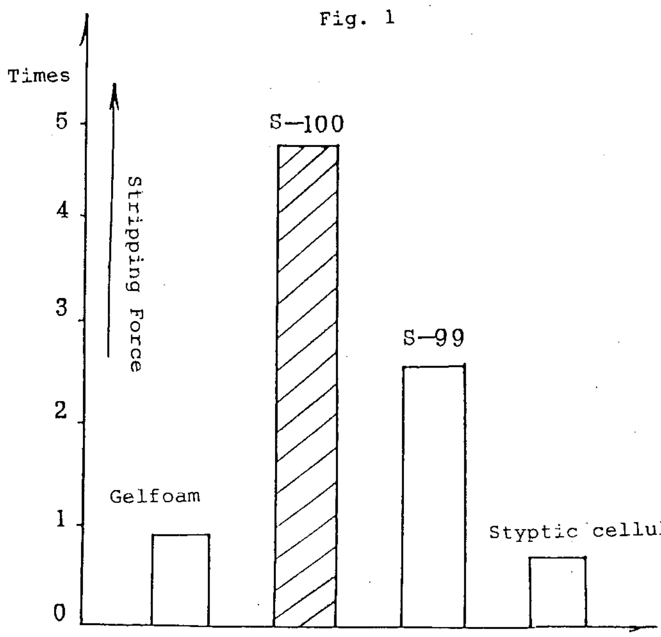




Rank Xerox (UK) Business Services

The present invention relates to soluble hemostatic fabrics and process for preparing them and their use in modern medicine. More particularly, the hemostatic fabrics of the present invention may dissolve or be absorbed by body fluid when contacting the bleeding wound. The hemostatic fabric is prepared by means of chemicals, using cellulosic fabric as starting material.

Thus far, the known hemostatic materials in the world usually can not dissolve when contacting the bleeding wound. Of these hemostatic materials generally there exist some shortcomings as follow while being used:

(1) no use for control of bleeding in vivo;
(2) not soluble on the bleeding wound;
(3) slow hemostatic speed;
(4) usually other hemostatic agent is added.

Therefore, it is necessary to develop the better hemostatic material desired in modern medicine and normal life of the human being. After a deliberate study of the inventor, the inventor fortunately and unexpectedly finds the soluble hemostatic fabric having Formula (I) and the soluble hemostatic fabric having Fomula (II),

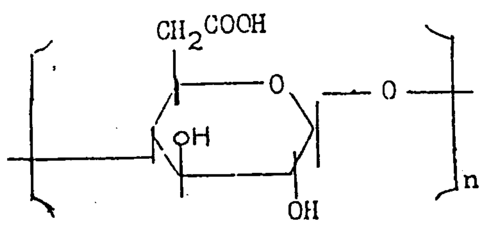

(I)

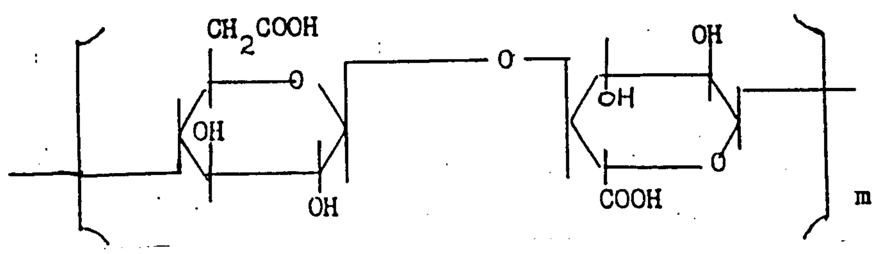

(II)

The two soluble hemostatic fabrics are obtained by treating cellulosic fabric by using chemical process. The two soluble hemostatic fabrics have the following advantages: dissoving at the bleeding wound in vivo or vitro; fast hemostatic speed; adding no other hemostatic agent; having good hemostatic effect for the patients who suffer from the distarbance of blood coagulation; leading to no occurance of any adverse reaction; easy to use, store and embody; good stability. Thus, the soluble hemostatic fabric of the invention may be widely used in any type of surgery and in other situations where the control of bleeding is required; they can also be economically prepared in industrial fields.

It is therefore an object of the present invention to provide satisfactory and secure hemostatic materials and a procers for preparing them. The inventor has carried out an extensive and deep study for this purpose for many years and finally found the soluble hemostatic fabrics having Formula (I) or Formula (II) which have excellent hemostatic effect on bleeding wound in vivo or vitro. Based on the above-mentioned discovery, the inventor has fulfilled the present invention.

Summary of the present invention

The first object of the present invention is to provide soluble hemostatic fabric having Formula (I) (hereafter refer as S-99), a process for preparing it and its use in modern madicine,

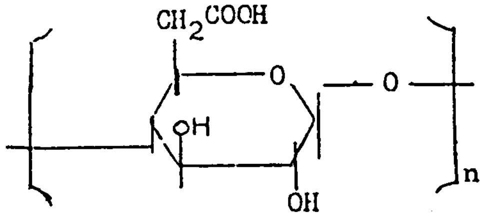

(I)

wherein n = 8,000-12,000.

It is known that the compound having Formula (I) is powder-like and is used as antiprecipitating agent of injection in pharmaceutical art. The S-99 ined from natural cotten cellulose treated by the chemical process of the present invention. The natural cotton cellulose has ability of absorbing water, but cannot dissolve in water. After having been treated by the chemical process of the invention, the physical and chemical fetures of the natural cotton cellulose product will greatly change so the fabric can dissobve in water. The soluble procedures of the S-99 at the bleeding wound is as follow: absorbing water-→expanding-→cellulosic tissue dispersion-→convertion into transparent colloid-→dissolution. The S-99 of the present invention may produce hemostatic effect by the following two channels:

1. physiologral channel:

The S-99 of the present invention can activate the coagulation factors and promate (accelerate) the formation of thrombins;

2. physical channel:

while contacting blood (including tissue fluid), S-99 absorbs it, swells up quickly and forms colloid, increases viscosity of blood, thus decreasing the speed of blood flow to block the end of the blood capillary.

As the S-99 of the present inventin may activate coagulation factors and accelerate the formation of thrombins in body, it has a good and quick control bleeding effect, especially in using for the patients who suffer from the disturbance of blood coagulation.

The S-99 is prepared by chemical method, which comprises:

a) treating the pure cotton fabric for 2-3 hrs with alkali metal hydroxide reactive solution (containing aqueous alkali metal hydroxide solution and lower $C_{1-4}$alkyl alcohol) at room temperature;

b) adding monohalogenated acetic reactive solution (containing monohalogenated acetic acid and lower $c_{1-4}$alkyl alcohol) to the pure cotton fabric of a) and stirring for 8-10 hrs at the same temperature;

c) adjusting pH of the fabric of b) to pH = 6.5-7.5 with hydrochloric acid and ethanol;

d) washing the fabric of c) with ethanol, until the Nacl amount in the fabric of c) is less than 1% by weight.

According to the present invention, the preferred method for preparing the S-99 of the present invention includes:

$a_1$) treating the pure cotton fabric for 2-3 hr with sodium hydroxide reactive solution (containing 45% aquous sodium hydroxide solution and 95% ethanol) at the room temperature;

$b_1$) adding monochloric acetic acid reactive solution (containing monochloracetic acid and 95% ethanol) to the pure catton fabric of a), and stirring for 6-10 hrs at the same temperature;

$c_1$) adjusting pH of the fabric of b) to pH = 6.5-7.5 with 20% hydrochloric acid and 75% ethanol;

$d_1$) washing the fabric of c) with 75% ethanol, until the Nacl amount in the fabric is less than 1% by weight .

The S-99 obtained according to the method of the present invention may cover the wound, and dissolve or be absorbed by body fluid, the S-99 is usually used for controling the bleeding in vitro.

Another object of the present invention is to provide a new soluble hemostatic fabric having formula (II) (hereafter refer as S-100), a process for preparing it and its use in modern medicine,

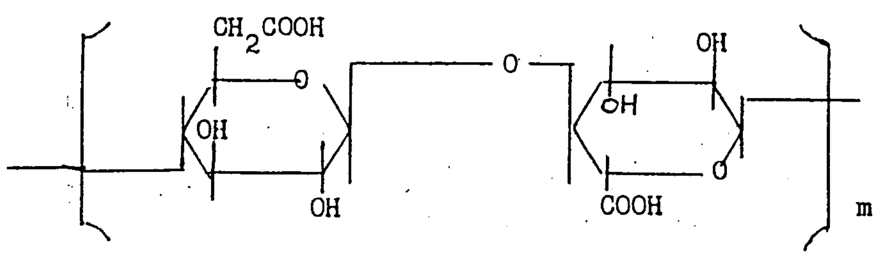

(II)

wherein m = 400-600

According to the present invention the S-100 is obtained by treating the high pure re-generated cellulose. Comparing with S-99, S-100 uses the obligue tissue fabric having the closed tissue as starting material, the S-100 obtained from the oblique tissue gabric has the following advantages, for example, high strength, good perfomrance to combine with the bleeing wound, lower and homogeneous polymerization degree etc. Therefore, while contacting the blood (including tissue fluid), S-100 not only may be soluble, but also is gradually degradated to form low molecular weight carbohydroate absorbed by human body. The soluble procedure of S-100 at the wound is the same as S-99, i.e, absorbing water-→expanding-→tissue dispersion-→convertion into transparent colloid-→full dissolution. The S-100 products have hemostatic effect at the wound by the following three channels:

1) physiological channel:

The S-100 may activate coagulation factors and acclerate the formation of thrombins in human body:

2) physical channel.

while contacting blood (including tissue fluid), the S-100 absorbs blood (including tissue fluid) and suells up quickly, forms colloid, increase viscosity of blood, thus decreasing the speed of blood so as to block the end of the blood capillary;

3) chemical channel:

The S-100 has the function of coagulating platelets.

As the S-100 may activate coagulation factors and promote the formation of thrombins in hyman body, the S-100 may also resulat in good hemostatic effect for the patients who suffer from the disturbance of blood coagulation.

Comparing with S-99, the S-100 has good physical and chemical homogenty and has broad applications, thus the S-100 may be used for controling the bleeding in vivo or vitro, and improves the hemostatic effect and speed. The S-100 is a much better hemostatic material than S-99. The S-100 having formula (II)

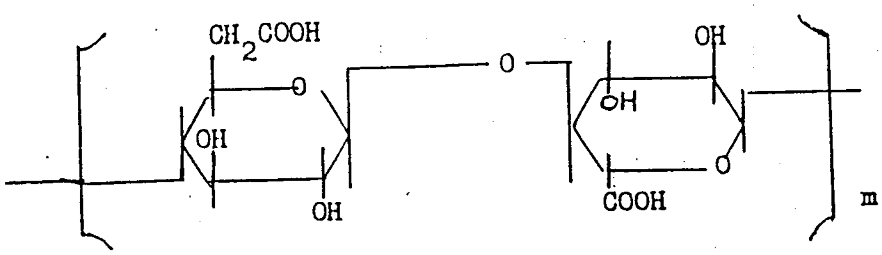

(II)

(wherein m = 400-600)

is prepared by means of a chemical process, which comprises:

a') at the room temperature, in the condition of pH = 9-10.5, treating the oblique fabric composed of the pure re-genated cellulose for 40-60 mins with sodium hypochlorite solution, and following the removal of the solution, washing it;

b') under the condition of 95-100 °C of the temperature and pH = 9-10.5, treating the fabric of a) 50-70 mins with hydrogen peroxide solution, and following the removal of hydrogen peroxide solution, washing the fabric with water:

c') at room temperature, treating the fabric of b) for 2-3 hrs with alkali metal hydroxide reactive solution as described in a);

d') treating the fabric of c) for 6-10 hrs with mono-halogenated acetic acid reactive solution as described in b);

e') adjusting pH of the fabric of d) to pH = 6.5-7.5 with ethanol and hydrochloric acid;

f') washing the fabric of e) with ethanol, until the Nacl amount in the obtained fabric is less than 1% by weight.

More particularly, the process for preparing the S-100 includes the following steps:

$a_1'$ ) under the conditions of the room temperature and the pH = 9-10.5, treating the oblique tissue fabric composed of the pure re-generated cellulose for 40-60 mins with sodium hypochlorite containing 1-4 gram effective chlorine/l and following the removal of sodium hydpochlorite, washing the fabric with water;

$b_1'$ ) under the conditions of the temperature = 95-100 °C, and pH = 9-10.5, treating the fabric of a) for 50-70 minutes with hydrogen peroxide soluton containing 1-5 grams hydrogen peroxide/l, and following the removal of hydrogen peroxide, washing with water.

$c_1'$ ) treating the fabric of b) for 2-3 hrs with sodium hydroxide solution (containing 45% aqueous sodium hydroxide solution and 95% ethanol);

$d_1'$ ) treating the fabric of c) for 6-10 hrs monochloric acetic acid reactive solution (containing monochloric acetic acid and 95% ethanol);

$e_1'$ ) adjusting the pH of the fabric of d) to pH=6.5-7.5 with 20% hydrochloric acid and 70-85% ethanol (perfenably 75% ethanol);

$f_1'$ ) washing the fabric of e) with 75% ethanol, until the Nacl amount in the fabric is less than 1% by weight.

The example for preparing the S-100 will be illustrated in detail hereafter.

The term "treating" in the present invention means that the fabric used in the present invention is stirred with the solution containing a compound used in the present invention.

The term "solubility" in the present invention means that the S-99 and the S-100 of the present invention may be sluble at the wound.

The following is to illustrate the physical and chemical features of the S-99 and the S-100 of the present invention:

1. solubility in water:

It is known that cellulosic cellulose contains a hydrophilic hydroxy. Although the cellulose has good absorbing-water ability, it is not soluble in water since there are a large amount of hydrogen bonds among its molecules and it has a higher crystallization degree.

However, after having been treated by chemical process of the present invention, the physical ad chemical features of the cellulose products change as below:

--when polymorization degree drops, dispersion force and induction force among cellulosic molecules alos decrease;

--as hydrophilic group is introduced, the spacing between molecules is enlarged, so the hydrorogen bond is destroyed;

--when crystallizatin degree decreases and the amorphous area increases, the orientation force decreases, so the hydrogen molecule has the possibity to get into the microcell to form molecular compound. Thus, the S-99 and the S-100 of the invention may be soluble in water.

2. adsorbability for water and polar medium:

The hemostatic meterial can improve hemostatic effect if a haemostatic material has good adsorbability for water and polar medium.

The adsorbability experiment of the invention is carried out as follow:

The S-99 and the S-100 of the present invention are respectively cut into 1 X 10 cm cloth strips, then, with these cloth strips put into the mixed soluton composed of 75% ethenol and 25% water, the ascending height of the liquid surface of these clothe strips is determined after five minutes. The result is as shown in Table I:

Table I

| Ascending Height (cm) | | |
|---|---|---|
| sample | longitudinal | latitudinal |
| S-99 | 2 | 1.5 |
| S-100 | 5 | 4.5 |

The data in Table I shows that the S-99 and the S-100 of the present invention have good absorbability for water or polar medium.

3. Combination ability for body surface

The ideal hemostatic material should have excellent combination ability for boby surface, especially the human skin.

The combination test for body surface in the invention is carried out by using the pure silk as the model of human skin. The S-99 and the S-100 of the invention and the gelfoam and the styptic are respectively cut into 5 X 10 cm size. Then place them in between two layers of the pure silk fabric, moisten them with the distilled water, dry them and finally dertermine the stripping force of them. In the combination ability test for body surface in the present invention, the stripping force of gelfoam is difined as 1, then S-100 is 5, S-99 is 2.7 and the styptic cellulose is 0.8. These results are as shown in figure 1. It is obvious from Figure 1 that the S-99 and the S-100 of the present invention have excellent

combination ability for body surface.

4. stability:

The S-99 and the S-100 of the present invention stored over two years at room temperature are compared with the S-99 and the S-100 newly prepared. The result show that the S-99 nd the S-100 stored over two years have the identical hemostatic effect as that of new S-99 and S-100. Therefore, the S-99 and the S-100 of the invention have stable chemical character and are easily to be stored and carried.

The description of hemostatic channels of S-99 and S-100.

1. physical channel:

while contacting (including tissue fluid), the S-99 and the S-100 of the invention with the wound, they will first absorb a great amount of the water content in the blood so as to increase the concentration and the viscosity of blood, and decrease the speed of blood flow; mean while, S-99 and S-100 swell up quickly and produce colloid after absorbing water, thus covering the wound and blocking the end of the blood capillary. Therefore the ability for absorbing water is important for a hemostatic material. The following test illustrates the ability of S-99 and S-100 for absorbing water:

The S-99, the S-100, the gelfoam and the styptic cellulose are respectively formulated into their 0.5% aqueous solution, then putting 1 X 10 cm filter paper into each solution bolow 1 cml, determining the ascending height of the liquid surface of the filter paper in each solution after five minutes, the results are measured adn summarised in Table 2.

Table 2

| Sample | S-100 | S-99 | gelfoam | styptic cellulose |
|---|---|---|---|---|
| ascending hight (cm) | 2 | 1.5 | 4.5 | 4.5 |

note: The larger the ascending height is, the lower the ability of a hemostatic material for absorbing water is. It is apparent from the Table 2 that S-99 and S-100 have good ability for absorbing water.

2. chemical channel:

The term "chemical channel" in the invention means that S-99 and S-100 may adsorb onto and coagulate the blood platelets in blood.

The platelet adsorption and coagulation test is carried out as follow:

pure cotton fabric, oxidized cellulosic fabric, gelfoam, styptic cellulose, S-99 ad S-100 (each weights:0.02g) are respectively put on the slide, then adding 0.04 ml blood treated by sodium citrate thereto, observing with the microscope, the following results are obtained and summarized in Figure 2. It is seen from Figure 2 that S-99 and S-100 have excellent adsorption and coagulation ability for platelets.

3 physiological channel:

The term "physiological channel" in the invention means that S-99 and S-100 may activate coagulation factors and accelerate the fomation of thrombins. The coagulation factors are the key factor to activate endogenous coagulation system and exogenous coagulation system. It is known that some coagulation factors have positive charge, they may be activated by the substances having negative charge. The S-99 and the S-100 of the invention may be soluble in water, they may product some negative charge after contacting water, therefore, they may activate coagulation factors.

The description of the hemostatic effect of S-99 and s-100:

The test of hemostatic effect of S-99 and S-100 is conducted on rabbit.

The bleeding models are respectively obtained from the liver, the kidney, the subdural space, the coaliac artery and the vein and the soft tissue of rabbit; for a same organ are prepared four same bleeding wounds, then controlling their bleedings with S-99, S-100, gelfoam and styptic cellulose, observing the time of stopping the bleeding and the bleeding amount, the following results are obtained and summarized in Figure 3 and Figure 4.

Figure 3 is the hemostatic time curve of each hemostatic material on each kind of organ.

Figure 4 is the bleeding amount curve for each hemostatic material on different kinds of organ when the

bleeding is blocked.

It is appearent from Figure 3 and Figure 4 that S-99 and S-100 are better hemostatic material than the ordinary hemostatic material.

It will be understood that the following specific examples are provided to illustration the present invention while not limiting the scope of the present invention.

Example 1

preparation of S-99

At room temperature, 30.0g pure cotton fabric is added to 500ml rotary reactor, adding 150ml sodium hdyroxide reactive solution (formulation: 45% aqueous sodium hydroxide solution (37.5 parts. v/v), 95% ethanol (62.5 parts. v/v) thererto, the obtained mixture is stirred for two hrs. Then, adding 90 ml monochloroacetic acid reactive solution (formulation: monochloroacetic acid, 40 parts, w/w; 95% ethanol, 60 parts, w/w) to the same reactor, said stirring is continued for 6 hours. The pH of the obained fabric is adjusted to pH = 6.5-7.5 with 20% hydrochloric acid solution then washing it with 75% ehtanol till the Nacl amunt in the obtained fabric being less than 1%, drying and sterilizing, the object product is achieved.

Example 2

At room temperature, 30.0g oblique tissue fabric composed of viscose cellulse is added to 500ml ratory reactor, 300ml sodium hydpochlorite solution is added thereto (effective chlorine: 2 g/l)at pH = 9-10.5 and stirred for 1 hour; then removing the sodium hypochlorite, washing it with water, The obtained fabric is taken out and placed again into the same reactor above disclosed under the condition of pH = 9-10.5 and at the temprature from 95 to 100 °C adding 300ml hydrogen peroxide solution (concentration: 0.3%) and 1.2g sodium pyrophosphate and stirring 1 hour, then removing the hydrogen peroxide solution, washing the obtained fabric with water until the added residual compounds will not be contained in the obtained fabric. Said fabric is then put again into the same reactor above mentioned, after adding 250 ml sodium hydroxide reactive solution [containing 45% aqueous solium hydroxide solution (37.5 parts, v/v) and 95% ethanol (67.5 parts,v/v)] thereto and stirrig 2 huors, then adding 90 ml monochloroacetic acid reactive solution (containing monochloroacetic acid (40 parts. w/w) ad 95% ethanol (60 parts, w/w)] thereto and continuing to stir for 6 hours, the obtained fabric is taken up from the reactor. Adjusting the pH thereof to pH = 6.5-7.5 with 20% hydrochloric acid and 75% ethanol, washing it with 75% ethanol untill the Nacl amount in the fabric becomes less than 1%, drying and sterilizing, the object product is finally obtained.

The principal advantages of the hemostatic materials obtained in accordance with the method of the present invention are that they can quickly produce hemostatic effect while contacting the wound, dissolve at the wound or be absorbed by body fluid without being added any hemostatic agent and leave in the closed wound no concommitant occurrence of any adverse reaction.

The hemostatic materials according to the present invention have broad application in any type of surgery and in other situations where the contril of bleeding is required, especially in the use for the patients who suffer from the disturbance of blood coagulation.

It will be apparent from the foregoing that the present invention provides a new hemostatic material having herebefore incomparable solubility at the wound, which may be prepared by a simple and economic process without the need to employ expensive reagents.

## Claims

**1.** A soluble haemostatic fabric having formula (I)

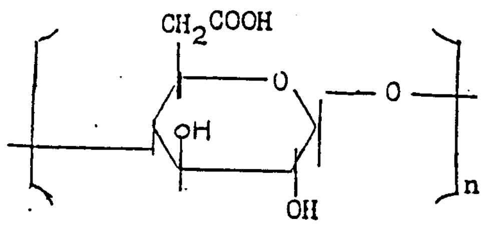

(I)

wherein n = 8,000-12,000.

**2.** A process for preparing a soluble haemostatic fabric having formula (I)

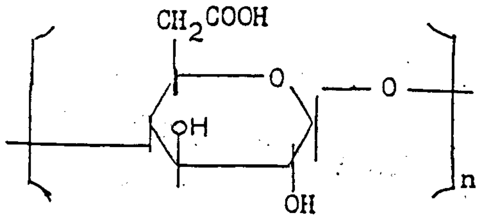

(I)

wherein n = 8,000-12,000,
which process comprises

a) treating a pure cotton fabric for 2-3 hours with an alkali metal hydroxide solution;
b) treating the fabric of a) for 6-10 hours with a monohologenated acetic acid solution;
c) adjusting the pH of the fabric of b) to pH = 6.5-7.5 with hydrochloric acid and 75%-85% ethanol;
d) washing the fabric of c) with 75-85% ethanol, until the NaCl amount in the fabric is less than 1% by weight.

**3.** A process according to claim 2, wherein the alkali metal hydroxide solution is composed of 45% aqueous sodium hydroxide and 95% $C_{1-4}$ alkyl alcohol.

**4.** A process according to claim 2 or 3, wherein the mono halogenated-acetic acid solution is composed of mono-chloroacetic acid and 95% $C_{1-4}$ alkyl alcohol.

**5.** A process according to claim 3 or 4, wherein the $C_{1-4}$ alkyl alcohol is ethanol.

**6.** A soluble haemostatic fabric having formula (II)

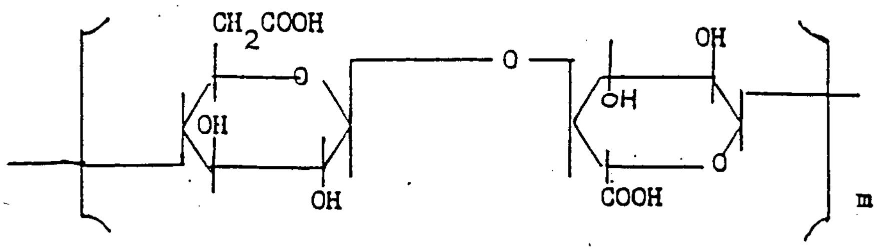

(II)

wherein m = 400-600.

**7.** A process for preparing a soluble haemostatic fabric having formula (II),

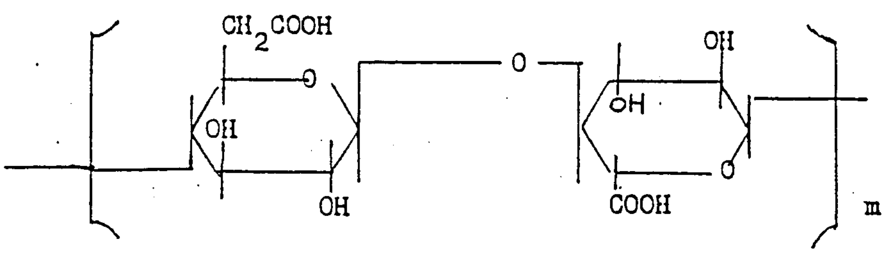

(II)

wherein m = 600-800,
which process comprises

a') treating an oblique tissue fabric composed of viscose cellulose with sodium hypochlorite solution, the treatment being performed for 40-60 minutes at room temperature and pH = 9-10.5,

b') treating at pH = 9-10.5 and 95-100 °C the fabric of a') for 50-70 minutes with 0.3% hydrogen peroxide solution and a small amount of sodium pyrophosphate,
c') washing the fabric of b') until substantially no sodium hydrochlorite remains in the fabric,
d') treating the fabric of c') for 2-3 hours with an alkali metal hydroxide solution,
e') treating the fabric of d') for 6-10 hours with a monohalogenated acetic acid solution,
f') adjusting the pH of the fabric of e') to pH = 6.5-7.5 with hydrochloric acid and 75-85% ethanol, and
g') washing the fabric of f') with 75% ethanol, until the NaCl amount in the fabric is less than 1% by weight.

**8.** A process according to claim 7, wherein the alkali metal hydroxide solution contains 45% aqueous sodium hydroxide solution and 95% $C_{1-4}$ alkyl alcohol.

**9.** A process according to claim 7 or 8, wherein the monohalogenated acetic acid solution is composed of monochloroacetic acid and 95% $C_{1-4}$ alkyl alcohol.

**10.** A process according to claim 7, 8 or 9, wherein the said $C_{1-4}$ alkyl alcohol is ethanol.

**11.** A process according to any one of claims 2 to 5 and 7 to 10, wherein the concentration of the hydrochloric acid is 20%.

Fig. 1

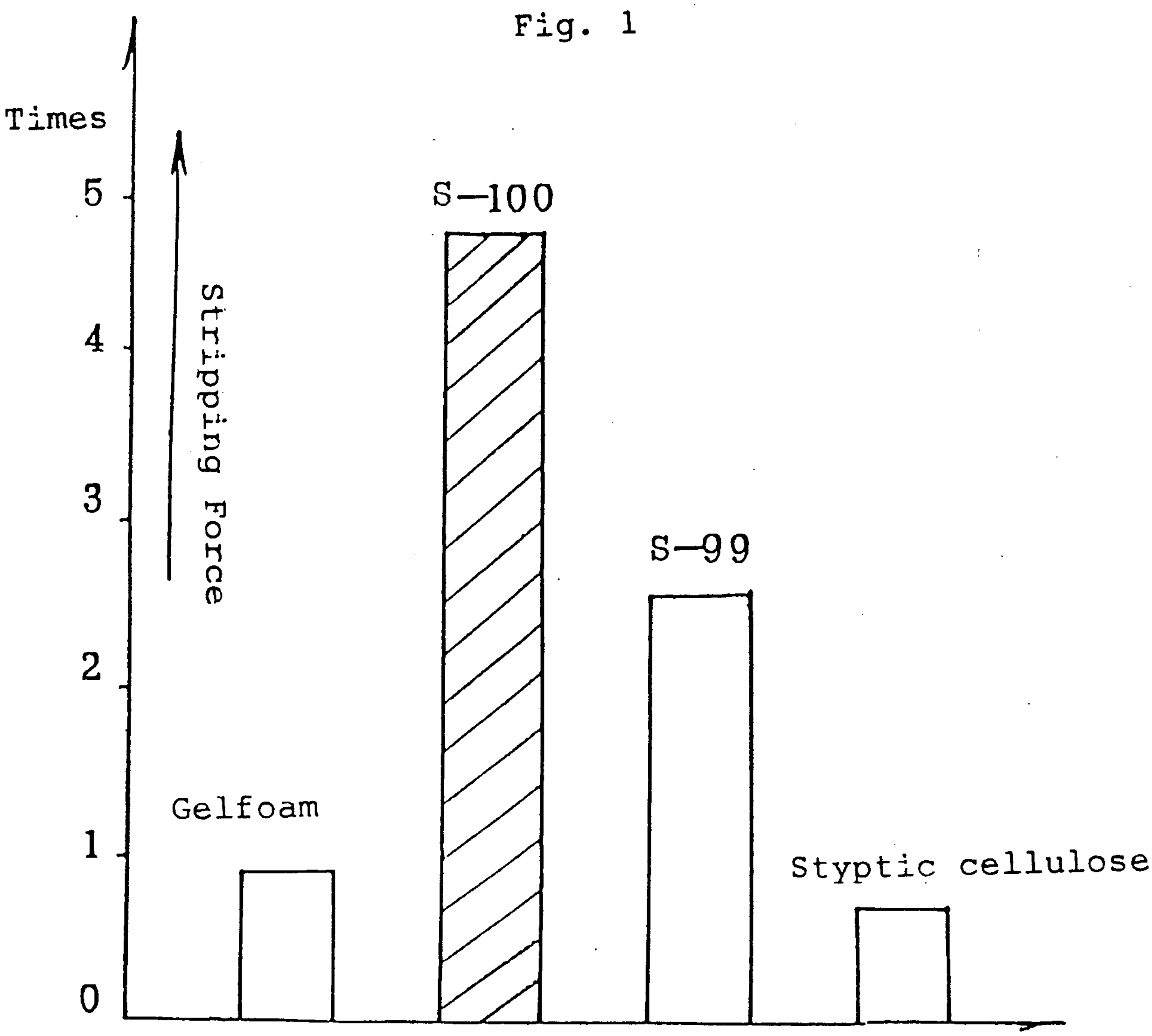
Times
5
4
3
2
1
0
Stripping Force
S—100
S—99
Gelfoam
Styptic cellulose

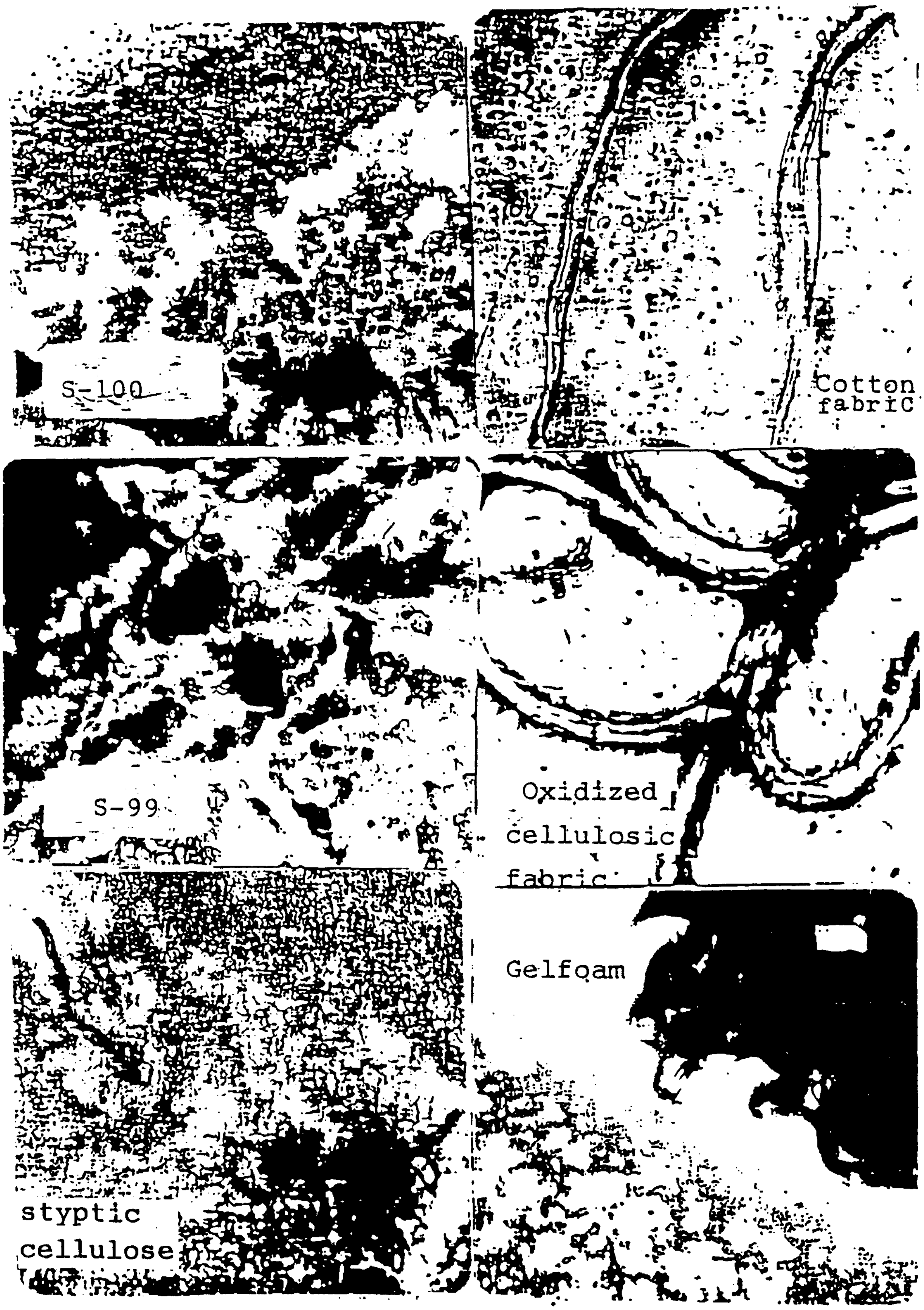
S-100
Cotton fabric
S-99
Oxidized cellulosic fabric
Gelfoam
styptic cellulose

Fig. 3

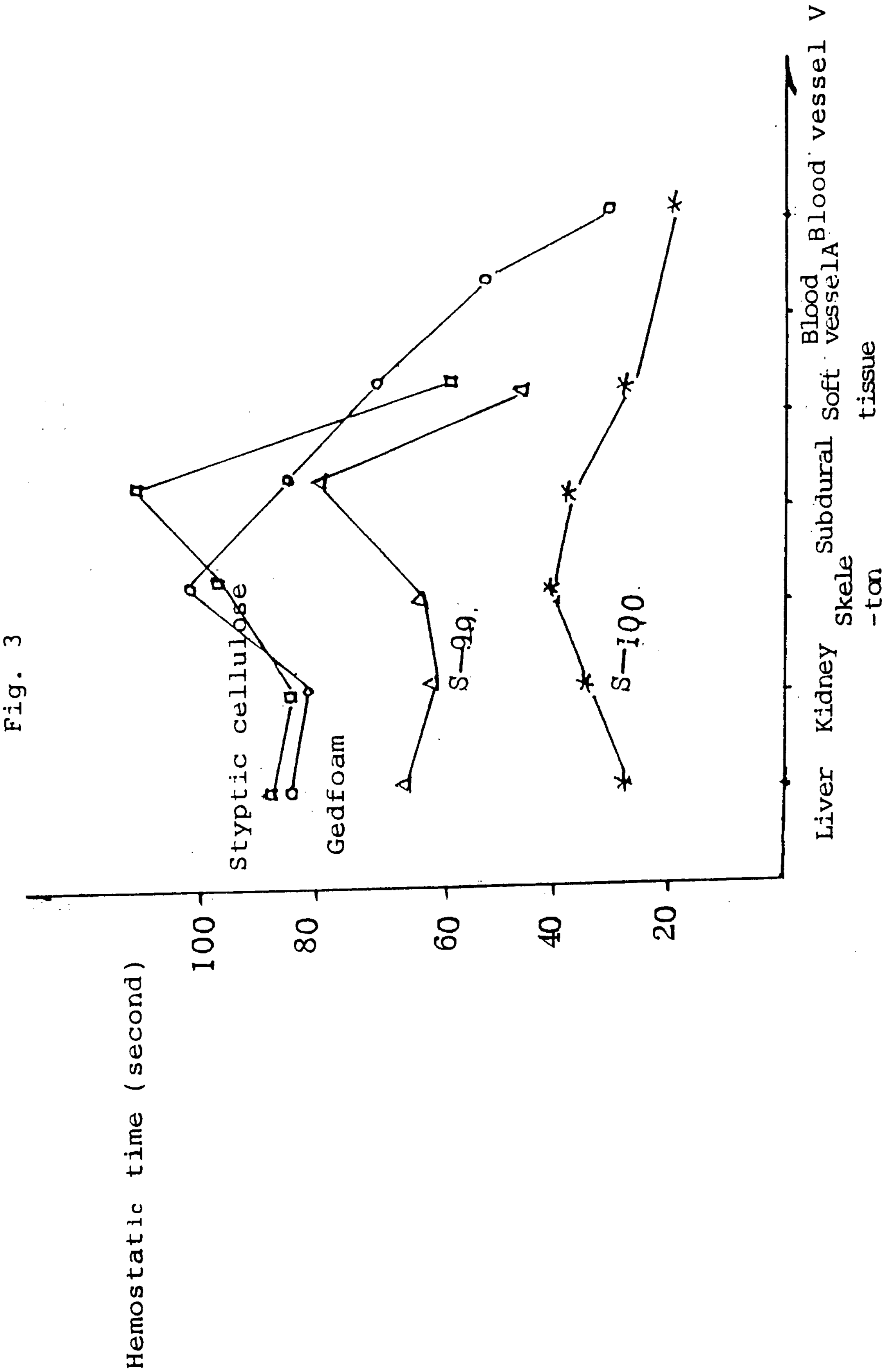
Hemostatic time (second)
100
80
60
40
20
Liver
Kidney
Skele-ton
Subdural
Soft tissue
Blood vessel A
Blood vessel V
Styptic cellulose
Gedfoam
S-99
S-100

Fig. 4

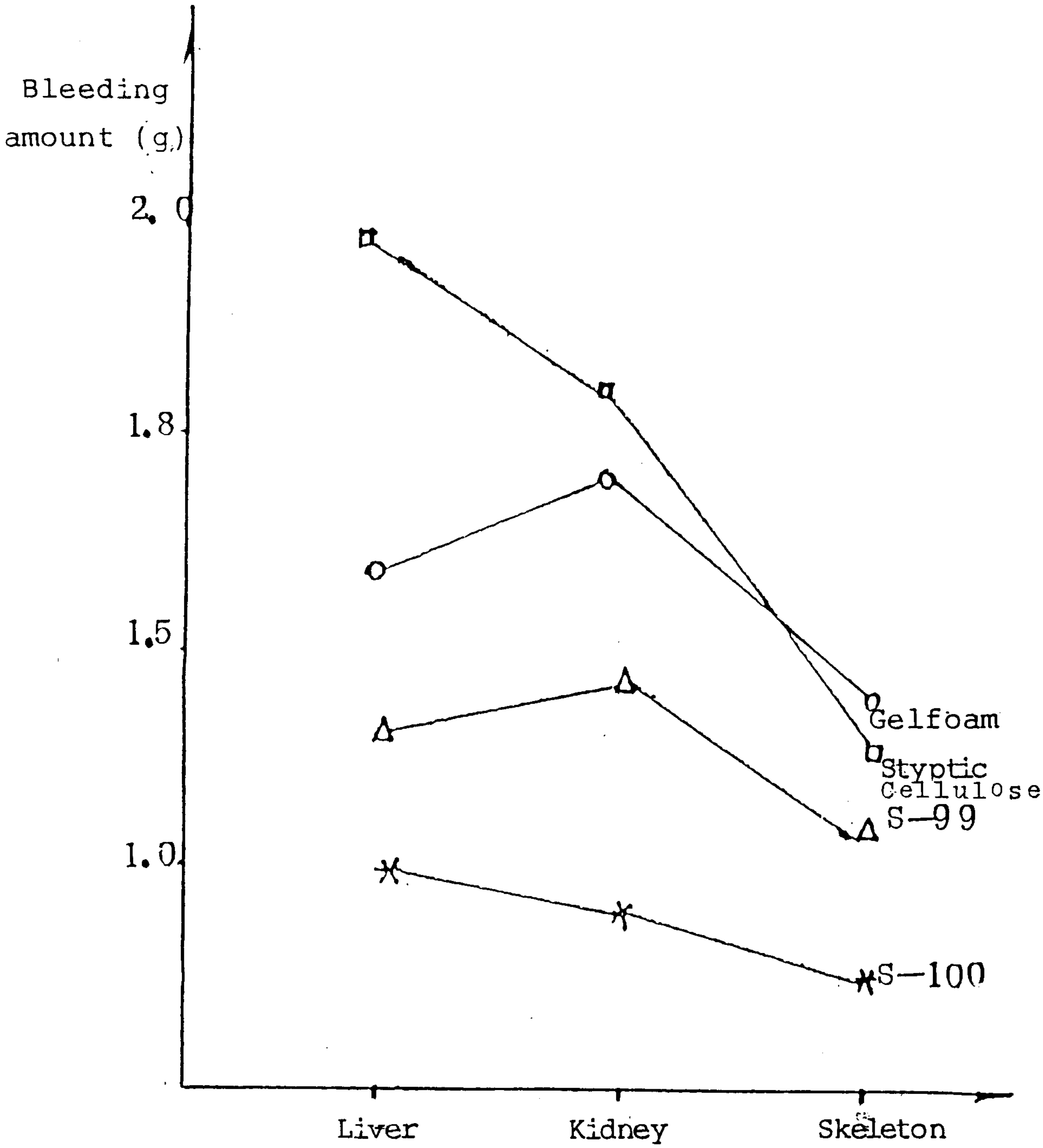
Bleeding amount (g)
2.0
1.8
1.5
1.0
Gelfoam
Styptic Cellulose
S—99
S—100
Liver
Kidney
Skeleton